# EUROPEAN PATENT APPLICATION

(11) **EP 3 722 293 A1**
(43) Date of publication of application: **14.10.2020**
(21) Application number: 18886529.9
(22) Date of filing: 05.12.2018
(51) Int. Cl.: C07D 405/14, A61K 31/4545, A61P 9/12, A61P 9/04

(54) **CRYSTAL FORM OF RENAL OUTER MEDULLARY POTASSIUM CHANNEL INHIBITOR AND PREPARATION METHOD THEREOF**

(30) Priority: 06.12.2017 CN 201711273099
(71) Applicant: Jiangsu Hengrui Medicine Co., Ltd., Lianyungang, Jiangsu 222047 (CN)
(72) Inventor: WU, Guaili, Lianyungang Jiangsu 222047 (CN); ZHANG, Quanliang, Lianyungang Jiangsu 222047 (CN); LU, Yun, Lianyungang Jiangsu 222047 (CN); YAO, Fei, Lianyungang Jiangsu 222047 (CN)
(74) Representative: dompatent von Kreisler Selting Werner - Partnerschaft von Patent- und Rechtsanwälten mbB
(86) International application number: PCT/CN2018/119310
(87) International publication number: WO 2019/109935

(57) **Abstract**

The present invention provides a crystal form of a renal outer medullary potassium channel inhibitor and a preparation method thereof. In particular, the present invention provides crystal form III of a L-tartrate of a renal outer medullary potassium channel (ROMK) inhibitor (I) and a preparation method thereof. The crystal form III has good chemical stability and crystal form stability, and the crystallization solvent used has low toxicity and residue. Thus, the present invention can be better used in clinical treatment.

## Description

The present claims the priority of Chinese patent application CN201711273099.2 filed on December 6th, 2017, the content of which is hereby incorporated by reference in its entirety.

### Field of invention

The present invention relates to a crystal form of renal outer medullary channel inhibitor and a preparation method thereof.

### Prior arts

Strengthening the salt reabsorption function of kidney will cause hypertension risk. On the contrary, inhibiting the reabsorption function of kidney can promote the excretion of urine and play a diuretic and antihypertensive role. Common diuretics include thiazide diuretic medicine, which are first-line antihypertensive drugs in the United States and mainly act on Na⁺-Cl transport carriers; Loop diuretics are more effective for patients with renal dysfunction, mainly through Na⁺-K⁺-2Cl-transporter. However, both kinds of the diuretics can cause hypokalemia (symptoms: weakness, fatigue, muscle spasm, constipation and cardiac rhythm problems such as arrhythmia), which increases the risk of morbidity and mortality of cardiovascular diseases.

The renal outer medullary potassium channel (ROMK) is also called inward-rectifying potassium channel 1.1 (KIR 1.1). ROMK ion channel can regulate Na⁺ reabsorption through the apical membrane conductance of the renal thick ascending limb (TAL) in coordination with Na⁺-K⁺-2Cl⁻ cotransporter NKCC2 (responsible for NaCl transport). Studies have found that ROMK is directly related to the secretory channel of kidney, after knocking out the ROMK gene, the 35-pS ion channel of the mouse TAL and CCD and other K⁺ ion channels of TAL are deleted. Batter syndrome is an autosomal recessive hereditary disease, characterized by a large amount of salt loss in the kidney, hypokalemia and low blood pressure. Parabulbar cell hyperplasia is mainly caused by mutations of ROMK or Na⁺-K⁺-2Cl⁻cotransporter, except that hypokalemia of parabulbar cell hyperplasia caused by ROMK mutation is greatly alleviated compared with that caused by mutations of Na⁺-K⁺-2Cl⁻ cotransporter. In a conclusion, inhibition of ROMK function can effectively inhibit the salt reabsorption function of Na⁺-K⁺-2Cl⁻ transporter, promote urine excretion, and achieve diuresis and antihypertensive effects without causing hypokalemia.

WO2016091042A1 (date of publication 2016.06.16) discloses an renal outer medullary potassium channel (ROMK) inhibitor, the chemical name of which is (R)-5-cyano-N-(1-(2-hydroxy-2-(4-methyl-1-carbonyl-1,3-dihydroisobenzofuran-5-yl)ethyl) piperidin-4-yl)-4-methoxypyridinecarboxamide. Compared with other ROMK inhibitors, the compound has additional polar groups, which reduces ClogP, improves hERG selectivity and increases safety on the basis of maintaining the activity of ROMK inhibitor, the structure of which is represented by formula (II):

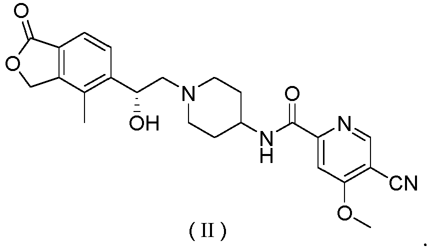

The crystal structure of the pharmaceutical active ingredient often affects the chemical and physical stability of the medicament. Different crystallization conditions and storage conditions may lead to changes in the crystal structure of the compound, sometimes be accompanied by the generation of other crystal forms. Generally speaking, amorphous drug products have no regular crystal structure and often have other defects, such as poor product stability, difficult filtration, easy agglomeration and poor fluidity. Therefore, it is necessary to improve various properties of the compound represented by formula (II).

### Content of the present invention

The technical problem to be solved by the present invention is to provide a crystal form III of (R)-5-cyano-N-(1-(2-hydroxy-2-(4-methyl-1-carbonyl-1,3-dihydroisobenzofuran-5-yl)ethyl)piperidin-4-yl)-4-methoxypyridinecarboxamide tartrate (represented by formula (I)) and a preparation method thereof, and the crystal form has good stability.

The technical solutions of the present invention are as follows:
The present invention provides a crystal form III of the compound represented by formula (I), wherein after Cu-Kα radiation, an X-ray powder diffraction spectrum represented by 2θ diffraction angles is obtained, in which there are characteristic peaks at 3.88, 7.54, 14.76, 18.64 and 22.21,

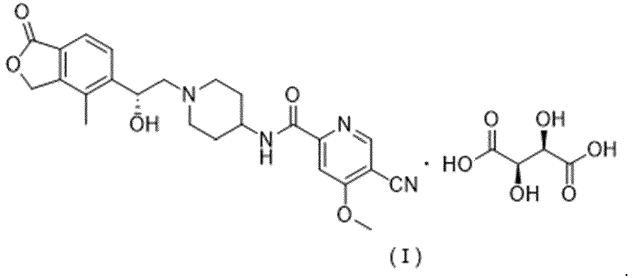

Preferably, the X-ray powder diffraction spectrum of crystal form III has characteristic peaks at 3.88, 7.54, 11.22, 14.76, 17.29, 18.64, 20.28, 22.21, 23.79, 25.34 and 27.09.

More preferably, the X-ray powder diffraction spectrum of crystal form III has characteristic peaks at 3.88, 7.54, 11.22, 11.61, 12.26, 12.73, 13.35, 13.64, 14.76, 15.98, 16.47, 17.07, 17.29, 18.64, 20.28, 20.62, 22.21, 23.16, 23.79, 24.14, 24.85, 25.34, 26.08, 26.85, 27.09, 28.77, 29.74, 32.22, 33.66, 34.50, 35.60, 37.42 and 39.27.

The present invention further provides a method for preparing crystal form III, which is one of the following methods:
Method (1): reacting the free state of the compound represented by formula (I) with L-tartaric acid in one solvent or a mixed solvent, then after stirring, crystallizing, filtering and drying, the target crystal form III is obtained; the solvent is a sulfoxide solvent, an amide solvent or an alcohol solvent, and the mixed solvent is a mixed solvent of a sulfoxide solvent and an alcohol solvent, or a mixed solvent of an amide solvent and an alcohol solvent; the sulfoxide solvent is preferably dimethyl sulfoxide, the amide solvent is preferably N,N-dimethylformamide or N,N-dimethylacetamide, and the alcohol solvent is preferably methanol, ethanol, *n*-propanol, *iso*-propanol or *n-*butanol;
Method (2): dissolving the compound represented by formula (I) in a solvent or a mixed solvent, then after crystallizing, filtering and drying, the target crystal form III is obtained; the crystallizing is conducted at room temperature, or while cooling or volatilizing the solvent or induced by crystal seed, and temperature for the cooling is -10 to 25°C, preferably the crystallizing is conducted at room temperature; the solvent is a sulfoxide solvent, an amide solvent or an alcohol solvent, and the mixed solvent is a mixed solvent of a sulfoxide solvent and an alcohol solvent, or a mixed solvent of an amide solvent and an alcohol solvent; the sulfoxide solvent is preferably dimethyl sulfoxide, the amide solvent is preferably N,N-dimethylformamide or N,N-dimethylacetamide, and the alcohol solvent is preferably methanol, ethanol, *n*-propanol, *iso*-propanol or *n*-butanol, the mixed solvent is more preferably dimethyl sulfoxide/methanol, dimethyl sulfoxide/ethanol, dimethyl sulfoxide/*n*-propanol, dimethyl sulfoxide/*iso*-propanol, dimethyl sulfoxide/*n*-butanol, N,N-dimethyl formamide/ethanol or N,N-dimethyl acetamide/ethanol.

The present invention further relates to a pharmaceutical composition of crystal form III comprising the crystal form III and a pharmaceutically acceptable carrier, a diluent or an excipient.

The present invention further relates to a method for preparing the pharmaceutical composition comprising mixing the crystal form III described above with the pharmaceutically acceptable carrier, the diluent or the excipient.

The present invention further relates to a use of the crystal form III or the pharmaceutical composition of crystal form III in the manufacture of a medicament for treating and/or preventing diseases or conditions related to renal outer medullary potassium channel (ROMK) inhibitors, wherein the diseases or conditions are preferably hypertension or heart failure. According to researches, compared with the free base form, the bioavailability and hygroscopicity of the crystal form III of the compound represented by formula (I) are improved: the bioavailability of the crystal form III in the mouse experiment (5mg/kg) is increased from 9283ng/mL x h of the free base to 12618ng/mL x h, and it can be seen that the *in vivo* bioavailability of the crystal form III is obviously better than that of the free base; on the other hand, compared with the free base, the crystal form III has a weight change of only 0.93% at 0%RH-80%RH, which has obvious advantages.

X-ray powder diffraction spectrum (XRPD) and differential scanning calorimetry (DSC) were used to determine the structure and study the crystal form III of the compound represented by formula (I).

The recrystallization method of crystal form III is not particularly limited and can be carried out referring to conventional recrystallization operations. For example, the raw material of the compound represented by formula (I) can be dissolved in an organic solvent and then added with an antisolvent for crystallization, and after the completion of the crystallization, the required crystal can be obtained through filtrating and drying.

The crystallizing in the present invention can be conducted while volatilizing the solvent, or at room temperature, or while cooling, or induced by crystal seed and the like.

The starting material used in the method for preparing crystal form of the present invention can be any form of the compound represented by formula (I), which includes but is not limited to amorphous or any crystal form and etc.

### Detailed description of the present invention

In the description and claims of the present application, unless otherwise specified, scientific and technical terms used herein have the meanings commonly understood by those skilled in the Art. However, in order to better understand the present invention, definitions and explanations of some relevant terms are provided below. In addition, when the definition and interpretation of the terms provided in the present application are inconsistent with the meaning commonly understood by those skilled in the art, the definition and interpretation of terms provided in the present application shall prevail.

The term "C₁₋₆ alkyl" used in the present invention represents for a linear or branched alkyl group having 1 to 6 carbon atoms, and specific examples include, but are not limited to, methyl, ethyl, *n*-propyl, *iso*-propyl, *n*-butyl, *iso*-butyl, *sec*-butyl, *tert*-butyl, *n*-pentyl, *iso*-pentyl, 2-methylbutyl, *neo*-pentyl, 1-ethylpropyl, *n*-hexyl, *iso*-hexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1,2-dimethylpropyl, and the like.

The term "alcohol solvent" used in the present invention refers to the solvent derived from substituting one or more than one hydrogen atoms on "C₁₋₆ alkyl" with one or more than one "hydroxyl (-OH)", the "C₁₋₆ alkyl" is as defined above, and specific examples include, but are not limited to, methanol, ethanol, *iso*-propanol, *n*-propanol, n-butanol, *iso*-pentanol or trifluoroethanol.

The "sulfoxide solvent" used in the present invention refers to a compound formed by combining thionyl (-SO-) and hydrocarbyl, and specific examples include, but are not limited to, dimethyl sulfoxide, diethyl sulfoxide or benzyl sulfoxide.

The "amide solvent" used in the present invention refers to a liquid compound in which the hydroxyl group of a carboxyl group contained in a carboxylic molecule is substituted by an amino group or a hydrocarbon amino group (-NHR or -NR₂); it can also be regarded as a liquid compound formed by substituting the hydrogen on a nitrogen atom contained in an ammonia or an amine molecule with an acyl; specific examples include, but are not limited to, N,N-dimethylformamide and N,N-dimethylacetamide.

The "mixed solvent" used in the present invention refers to a solvent obtained by mixing one or more than one different kinds of organic solvents in a certain ratio; the mixed solvent is preferably a mixed solvent of a sulfoxide solvent and an alcohol solvent, or a mixed solvent of an amide solvent and an alcohol solvent; the sulfoxide solvent is preferably dimethyl sulfoxide, the amide solvent is preferably N,N-dimethylformamide or N,N-dimethylacetamide, the alcohol solvent is preferably methanol, ethanol, *n*-propanol, *iso*-propanol or *n*-butanol, more preferably dimethyl sulfoxide/methanol, dimethyl sulfoxide/ethanol, dimethyl sulfoxide/*n*-propanol, dimethyl sulfoxide/*iso*-propanol, dimethyl sulfoxide/*n*-butanol, N,N-dimethyl formamide/ethanol or N,N-dimethyl acetamide/ethanol, the certain ratio can be volume ratio or mass ratio, the volume ratio is 0.05:1-1: 0.05, preferably 1:1, 1:2, 2:1, 4:1, 5:1 or 10:1, and the mass ratio is 10:1-1:10, preferably 5: 1, 2:1, 1:2 or 1.6:1;.

The "X-ray powder diffraction spectrum or XRPD" used in the present invention refers to that according to Bragg formula 2d sin θ = nλ (in the formula, λ is the wavelength of the X-ray, λ = 1.54056 Å, the number of the diffraction order n is any positive integer, generally taking the first-order diffraction peak, n=1), when X-ray is incident to an atomic plane having D lattice plane spacing of a crystal or part of a crystal sample at a grazing angle θ (the residual angle of an incident angle, also known as Bragg angle), the Bragg equation can be then satisfied, thus this group of X-ray powder diffraction patterns can be measured.

The "differential scanning calorimetry analysis or DSC" used in the present invention refers to measuring the temperature difference and heat flow difference between the sample and the reference substance in the process of heating or constant temperature of the sample, in order to characterize all physical and chemical changes related to thermal effect and obtain the phase change information of the sample.

The "2θ or 2θ angle" used in the present invention refers to diffraction angle, θ is the Bragg angle, the unit is ° or degree, and the error range of 2θ is ±0.1-±0.5, preferably ±0.1-±0.3, more preferably ±0.2.

The "crystal plane spacing or crystal plane spacing (d value)" used in the present invention refers to 3 unit vectors a, b and c selected from the space lattice that are not parallel and connecting the two adjacent lattice points, which divide the lattice into juxtaposed parallelepiped units, that is called crystal plane spacing. The spatial lattice is divided into a set of linear lattices, called spatial lattices or lattices, according to the determined parallelepiped unit lines. The dot matrix and lattice reflect the periodicity of the crystal structure with geometric points and lines respectively, different crystal planes have different surface spacing (i.e. the distance between two adjacent parallel crystal planes); the unit is Å or angstrom.

The present invention also relates to a pharmaceutical composition comprising crystal form III of the compound represented by formula (I), and optionally one or more than one pharmaceutical carrier and/or diluent. The pharmaceutical composition can be formulated into any pharmaceutically acceptable dosage form. For example, the crystal form III or the pharmaceutical preparation of the compound represented by formula (I) of the present invention can be formulated into tablets, capsules, pills, granules, solutions, suspensions, syrups, injections (including injection, sterile powder for injection and concentrated solution for injection), suppositories, inhalants or sprays.

In addition, the pharmaceutical composition of the present invention can also be subjected to patients or subjects in need of such treatment in any suitable administration, such as oral, parenteral, rectal, pulmonary or local administration, etc. When used for oral administration, the pharmaceutical composition can be made into oral preparations, such as oral solid preparations, such as tablets, capsules, pills, granules etc.; or, oral liquid preparations, such as oral solutions, oral suspensions, syrups, etc. When formulated into an oral preparation, the pharmaceutical preparation may also contain suitable fillers, binders, disintegrants, lubricants, etc. When used for parenteral administration, the pharmaceutical preparation can be made into injections, including injection liquid, sterile powder for injection and concentrated solution for injection. When prepared into an injection formulations, the pharmaceutical composition can be produced according to conventional methods in the existing pharmaceutical field. When prepared into an injection, there could be no additives added to the pharmaceutical preparation, and appropriate additives can also be added according to the nature of the drug. When used for rectal administration, the pharmaceutical preparation can be formulated into suppository, etc. When used for pulmonary administration, the pharmaceutical preparation can be made into inhalant or spray, etc. In some preferred embodiments, crystal form III of the compound represented by formula (I) of the present invention is present in a pharmaceutical composition or a drug in a therapeutically and/or prophylactically effective amount. In some preferred embodiments, crystal form III of the compound represented by formula (I) of the present invention is present in the pharmaceutical composition or drug in unit dose form.

The compound of the formula (I) and the crystal form III thereof can be used in the manufacturing of a medicament for treating a disease or a condition related to renal outer medullary potassium channel (ROMK) inhibition. Therefore, the present application also relates to a use of the crystal form III of the compound represented by formula (I) in manufacturing a medicament, the medication is used for treating the disease related to renal outer medullary potassium channel (ROMK) inhibition. In addition, the application also relates to a method for inhibiting a disease related to renal outer medullary potassium channel (ROMK) inhibition comprising administering to a subject in need thereof a therapeutically and/or prophylactically effective amount of the crystal III of the compound represented by formula (I) of the present invention, or the pharmaceutical composition of the present invention.

In some preferred embodiments, the disease is related to renal outer medullary potassium channel (ROMK) inhibition, which refers to hypertension or heart failure.

### The beneficial effects of the present invention

Compared with the prior art, the technical solution of the present invention has the following advantages:
Studies show that the crystal form III of the compound represented by formula (I) prepared by the present invention has good solubility and higher purity, and the crystal form detected by XRPD stays the same under the conditions of high temperature, high humidity and illumination, which illustrates good crystal form stability; the crystal form III of the compound represented by formula (I) obtained by the technical solution of the present invention can meet the medical requirements of production, transportation and storage, has stable, repeatable and controllable production process, which is suitable for industrial production.

### Brief description of drawings

Fig. 1 is an XRPD spectrum of the crystal form III of the compound represented by formula (I).
Fig. 2 is a DSC pattern of the crystal form III of the compound represented by formula (I).
Fig. 3 is an XRPD spectrum of the crystal form I of the compound represented by formula (I).
Fig. 4 is an XRPD spectrum of the crystal form II of the compound represented by formula (I).

### Detailed description of the preferred embodiments

The present invention will be explained in more detail below with reference to embodiments, which are only used to illustrate the technical solutions of the present invention, and do not limit the essence and scope of the present invention.

### Testing apparatus for experiments

### 1. DSC pattern

Apparatus model: MettlerToledo DSC 1 Staree System
Purge gas: nitrogen
Heating rate: 10.0 °C/min
Temperature range: 40-350 °C

### 2. X-ray diffraction spectrum

Apparatus model: Bruker D8 Focus X-ray powder diffractometer
Ray: Monochrome Cu-Kα Ray (λ=1.5406)
Scan mode: θ/2θ, scan range: 2-40°
Voltage: 40KV, Current: 40mA

The compound represented by formula (II) (free state) is prepared referring to the method described in the patent application WO2016091042A1 (publication date 2016.06.16).

### Comparative Example 1: Preparation of crystal Form I

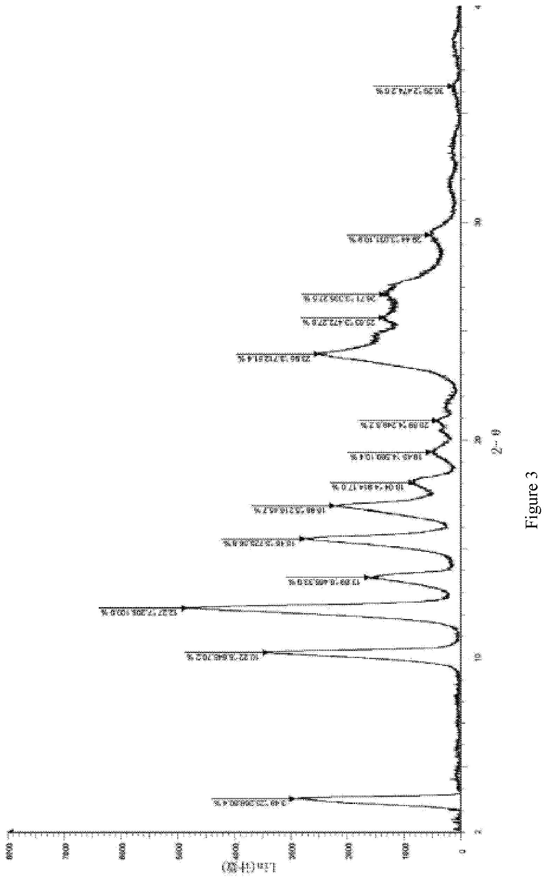

L-tartaric acid (0.4g, 2.66mmol) was added into a 50ml reaction flask, 30ml methanol was added, then the mixture was heated to 70°C until dissolving, and (R)-5-cyano-N-(1-(2-hydroxy-2-(4-methyl-1-carbonyl-1,3-dihydroisobenzofuran-5-yl)ethyl)piperidin-4-yl)-4-methoxypyridinecarboxamide (1.0g, 2.22mmol) (prepared according to the method described in patent application WO2016091042A1 (publication date 2016.06.16)) was added, then the mixture was reacted at 70°C for 24 hours, cooled to room temperature, followed by suction filtration and drying to obtain 1.22g solid, with a yield of 91.7%. The X-ray powder diffraction spectrum was shown in fig. 3, and the crystal form was defined as crystal form I.

### Comparative Example 2: Preparation of crystal Form II

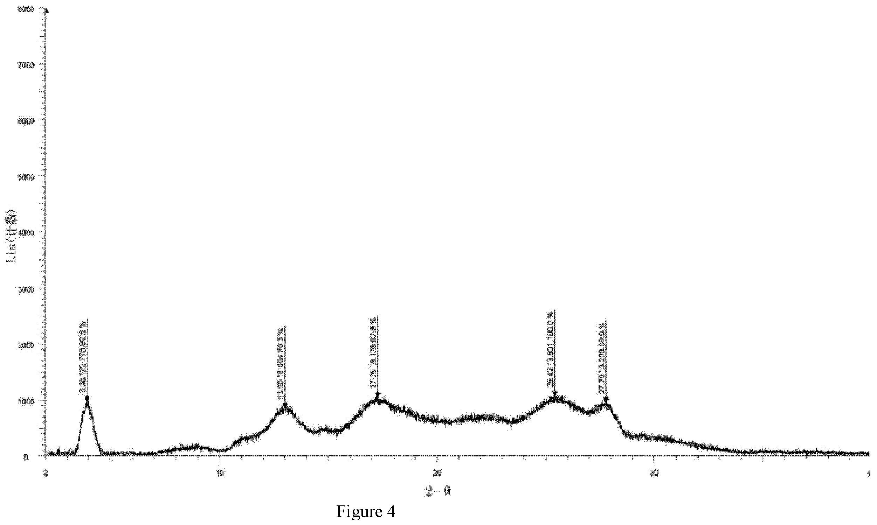

(R)-5-cyano-N-(1-(2-hydroxy-2-(4-methyl-1-carbonyl-1,3-dihydroisobenzofuran-5-yl)ethyl)piperidin-4-yl)-4-methoxypyridinecarboxamide (1.0g, 2.22mmol) (prepared according to the method in patent application WO2016091042A1 (publication date 2016.06.16)), L-tartaric acid (0.4g, 2.66mmol) were added into a 50ml reaction flask, 30ml *iso*-propanol/tetrahydrofuran/water (V:V:V=20:10:1) was added, the mixture was heated to 70°C for reaction for 24h, and then reduced to room temperature, followed by suction filtration and drying to obtain 1.15g solid, with a yield of 86.5%. The X-ray powder diffraction spectrum was shown in fig. 4, and the crystal form was defined as crystal form II.

### Embodiment 1 Preparation of crystal Form III

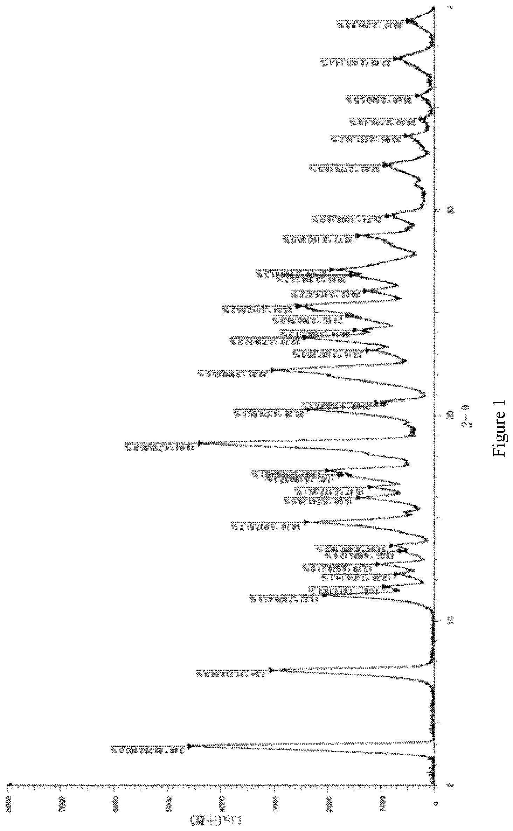

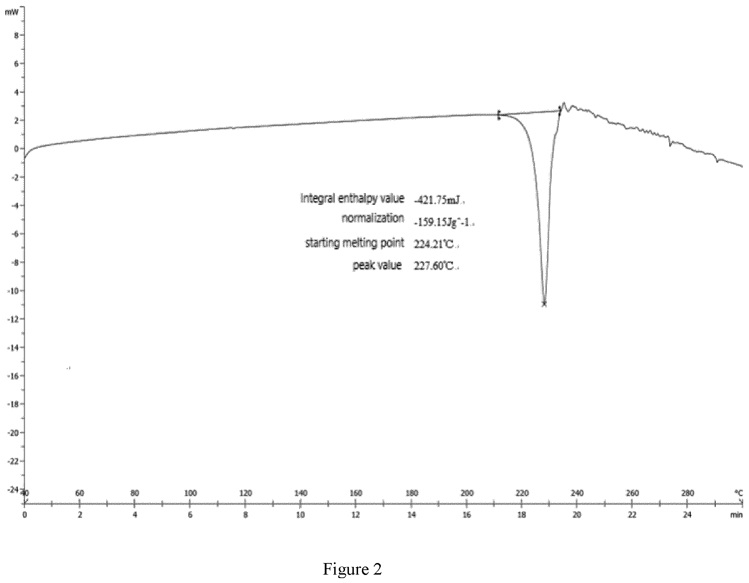

L-tartaric acid (0.4g, 2.66mmol) was added into a reaction flask, 25mL ethanol was added, the mixture was heated to 70°C until dissolving, and (R)-5-cyano-N-(1-(2-hydroxy-2-(4-methyl-1-carbonyl-1,3-dihydroisobenzofuran-5-yl)ethyl)piperidin-4-yl)-4-methoxypyridinecarboxamide (1.0g, 2.22mmol) (prepared according to the method described in patent application WO2016091042A1 (publication date 2016.06.16)) was added, the mixture was reacted at 70°C for 24 hours, then cooled to room temperature, followed by suction filtration to obtain 1.23g solid with a yield of 92.3%. The X-ray powder diffraction spectrum (XRPD spectrum) of the crystal sample was shown in Figure 1 and the DSC spectrum was shown in Figure 2, a sharp melting endothermic peak presents at 227.60°C, this crystal form was defined as crystal form III, and the 2θ characteristic peaks position as follows:

**Table 1, characteristic peaks of crystal form III**

| Peak number | 2θ[°] | d[Å] |
|---|---|---|
| Peak 1 | 3.88 | 22.75 |
| Peak 2 | 7.54 | 11.71 |
| Peak 3 | 11.22 | 7.88 |
| Peak 4 | 11.61 | 7.62 |
| Peak 5 | 12.26 | 7.21 |
| Peak 6 | 12.73 | 6.95 |
| Peak 7 | 13.35 | 6.63 |
| Peak 8 | 13.64 | 6.49 |
| Peak 9 | 14.76 | 6.00 |
| Peak 10 | 15.98 | 5.54 |
| Peak 11 | 16.47 | 5.38 |
| Peak 12 | 17.07 | 5.19 |
| Peak 13 | 17.29 | 5.13 |
| Peak 14 | 18.64 | 4.76 |
| Peak 15 | 20.28 | 4.38 |
| Peak 16 | 20.62 | 4.31 |
| Peak 17 | 22.21 | 4.00 |
| Peak 18 | 23.16 | 3.84 |
| Peak 19 | 23.79 | 3.74 |
| Peak 20 | 24.14 | 3.68 |
| Peak 21 | 24.85 | 3.58 |
| Peak 22 | 25.34 | 3.51 |
| Peak 23 | 26.08 | 3.41 |
| Peak 24 | 26.85 | 3.32 |
| Peak 25 | 27.09 | 3.29 |
| Peak 26 | 28.77 | 3.10 |
| Peak 27 | 29.74 | 3.00 |
| Peak 28 | 32.22 | 2.78 |
| Peak 29 | 33.66 | 2.66 |
| Peak 30 | 34.50 | 2.60 |
| Peak 31 | 35.60 | 2.52 |
| Peak 32 | 37.42 | 2.40 |
| Peak 33 | 39.27 | 2.29 |

### Embodiment 2 Preparation of crystal Form III

(R)-5-cyano-N-(1-(2-hydroxy-2-(4-methyl-1-carbonyl-1,3-dihydroisobenzofuran-5-yl)ethyl)piperidin-4-yl)-4-methoxypyridinecarboxamide (1.0g, 2.22mmol) (prepared according to the method described in patent application WO2016091042A1 (publication date 2016.06.16)) was added to a reaction flask A, and 10mL dimethyl sulfoxide was added, the mixture was heated to 70°C until dissolving, L-tartaric acid (0.4g, 2.66mmol) was added into a reaction flask B, 20mL anhydrous ethanol was added, the mixture was heated to 70°C until dissolving, then the clear solution in the reaction flask B was added into the reaction flask A, the reaction was carried out at 70°C for 4 hours, then cooled to room temperature, and stirred overnight. After suction filtration and drying, 1.13g solid was obtained with a yield of 85.0%. The X-ray powder diffraction spectrum and DSC pattern of the product were studied and compared, and the product was confirmed to be crystal form III.

### Embodiment 3 Preparation of crystal Form III

(R)-5-cyano-N-(1-(2-hydroxy-2-(4-methyl-1-carbonyl-1,3-dihydroisobenzofuran-5-yl)ethyl)piperidin-4-yl)-4-methoxypyridinecarboxamide (1.0g, 2.22mmol) (prepared according to the method described in patent application WO2016091042A1 (publication date 2016.06.16)) was added to a reaction flask A, and 10 ml N,N-dimethyl formamide was added, then the mixture was heated to 70°C until dissolving, and L-tartaric acid (0.4g, 2.66mmol) was added to a reaction flask B, 20mL anhydrous ethanol was added, the mixture was heated to 70°C until dissolving, the clear solution in the reaction flask B was added into the reaction flask A, the reaction was carried out at 70°C for 4 hours, then cooled to room temperature and stirred overnight. After suction filtration and drying, 1.10g solid was obtained with a yield of 82.7%. The X-ray powder diffraction spectrum and DSC pattern of the product were studied and compared, and the product was confirmed to be crystal form III.

### Embodiment 4 Preparation of crystal Form III

(R)-5-cyano-N-(1-(2-hydroxy-2-(4-methyl-1-carbonyl-1,3-dihydroisobenzofuran-5-yl)ethyl)piperidin-4-yl)-4-methoxypyridinecarboxamide (1.0g, 2.22mmol) (prepared according to the method described in patent application WO2016091042A1 (publication date 2016.06.16)) was added to a reaction flask A, and 10 ml N,N-dimethyl acetamide was added and the mixture was heated to 70°C until dissolving, and L-tartaric acid (0.4g, 2.66 mmol) was added to a reaction flask B, 20mL anhydrous ethanol was added and the mixture was heated to 70°C until dissolving, the clear solution in the reaction flask B was added into the reaction flask A, the reaction was carried out at 70°C for 4 hours, then cooled to room temperature and stirred overnight. After suction filtration and drying, 1.12g solid was obtained with a yield of 84.2%. The X-ray powder diffraction spectrum and DSC pattern of the product were studied and compared, and the product was confirmed to be crystal form III.

### Embodiment 5 Preparation of crystal Form III

The compound represented by the formula (I) (1.0g, 1.67mmol) (prepared according to Embodiment 1) was added into a reaction flask, 6ml N,N-dimethyl formamide was added, the mixture was stirred and dissolved at 70°C, then 12mL preheated anhydrous ethanol was added, the reaction was carried out at 70°C for 4 hours, then cooled to room temperature, and stirred overnight. After suction filtration and drying, 816mg solid was obtained with a yield of 81.6%. The X-ray powder diffraction spectrum and DSC pattern of the product were studied and compared, and the product was confirmed to be crystal form III.

### Embodiment 6 Preparation of crystal Form III

The compound represented by the formula (I) (1.0g, 1.67mmol) (prepared according to Embodiment 1) was added into a reaction flask, 6ml N,N-dimethyl acetamide was added, the mixture was stirred and dissolved at 70°C, then 12mL preheated anhydrous ethanol was added, the reaction was carried out at 70°C for 4 hours, then cooled to room temperature, and stirred overnight. After suction filtration and drying, 828mg solid was obtained with a yield of 82.8%. The X-ray powder diffraction spectrum and DSC pattern of the product were studied and compared, and the product was confirmed to be crystal form III.

### Embodiment 7 Preparation of crystal Form III

The compound represented by the formula (I) (1.0g, 1.67mmol) (prepared according to Embodiment 1) was added into a reaction flask, 5mL dimethyl sulfoxide was added, the mixture was stirred and dissolved at 70°C, then 10mL preheated methanol was added, the reaction was carried out at 70°C for 4 hours, then cooled to room temperature and stirred overnight. After suction filtration and drying, 794mg solid was obtained with a yield of 79.4%. The X-ray powder diffraction spectrum and DSC pattern of the product were studied and compared, and the product was confirmed to be crystal form III.

### Embodiment 8 Preparation of crystal Form III

The compound represented by the formula (I) (1.0g, 1.67mmol) (prepared according to Embodiment 1) was added into a reaction flask, 5mL dimethyl sulfoxide was added, the mixture was stirred and dissolved at 70°C, then 10mL preheated anhydrous ethanol was added, the reaction was carried out at 70°C for 4 hours, then cooled to room temperature, and stirred overnight. After suction filtration and drying, 864mg solid was obtained with a yield of 86.4%. The X-ray powder diffraction spectrum and DSC pattern of the product were studied and compared, and the product was confirmed to be crystal form III.

### Embodiment 9 Preparation of crystal Form III

The compound represented by the formula (I) (1.0g, 1.67mmol) (prepared according to Embodiment 1) was added into a reaction flask, 5mL dimethyl sulfoxide was added, the mixture was stirred and dissolved at 70°C, then 10mL preheated *iso-*propanol was added, the reaction was carried out at 70°C for 4 hours, then cooled to room temperature, and stirred overnight. After suction filtration and drying, 906mg solid was obtained with a yield of 90.6%. The X-ray powder diffraction spectrum and DSC pattern of the product were studied and compared, and the product was confirmed to be crystal form III.

### Embodiment 10 Preparation of crystal Form III

The compound represented by formula (I) (1.0g, 1.67mmol) (prepared according to Embodiment 1) was added into a reaction flask, 5mL dimethyl sulfoxide was added, the mixture was stirred and dissolved at 70°C, then 10mL preheated n-butanol was added, the reaction was carried out at 70°C for 4 hours, then cooled to room temperature, and stirred overnight. After suction filtration and drying, 896mg solid was obtained with a yield of 89.6%. The X-ray powder diffraction spectrum and DSC pattern of the product were studied and compared, and the product was confirmed to be crystal form III.

### Embodiment 11 Investigation of crystal Form Stability

The crystal form III of the compound represented by formula (I) obtained in Embodiment 1, and the samples of crystal form I and II obtained in Comparative Examples 1 and 2 were respectively placed open and evenly spread, and the stability of the samples under the conditions of illumination (4500Lux), heating (40°C, 60°C), and high humidity (75% RH, 90% RH) was investigated. The sampling time was 5 days and 10 days, and the purity was detected by HPLC.

### Experimental results:

**Table 2, Stability comparison of the samples of crystal forms I, II and III of the compound represented by formula (I)**

| Sample name | Time (days) | Illumination | 40°C | 60°C | 75% RH | 90% RH |
|---|---|---|---|---|---|---|
| Crystal form I | 0 | 99.67% | 99.67% | 99.67% | 99.67% | 99.67% |
| | 5 | 99.54% | 99.52% | 99.55% | 99.53% | 99.59% |
| | 10 | 99.34% | 99.45% | 99.39% | 99.39% | 99.43% |
| Crystal form II | 0 | 99.47% | 99.47% | 99.47% | 99.47% | 99.47% |
| | 5 | 99.23% | 99.38% | 99.22% | 99.30% | 99.30% |
| | 10 | 98.62% | 99.15% | 98.87% | 99.10% | 99.10% |
| Crystal form III | 0 | 99.65% | 99.65% | 99.65% | 99.65% | 99.65% |
| | 5 | 99.65% | 99.66% | 99.67% | 99.69% | 99.69% |
| | 10 | 99.57% | 99.66% | 99.64% | 99.66% | 99.66% |

### Experimental conclusion:

Stability investigation results show that when the samples of crystal forms I, II and III of the compound represented by formula (I) were placed respectively under open conditions, the HPLC purity of crystal form III was higher than that of crystal forms I and II under the conditions of illumination, high humidity and high temperature, indicating that the stability of the crystal form III of the present invention is better than the crystal forms I and II.

### Embodiment 13 Investigation on Special Stability of crystal Form

The crystal form III obtained in Embodiment 1 was ground, heated and tableted to investigate the stability of the sample.

### Experimental results:

**Table 3. Study on special stability of the crystal form III of the compound represented by formula (I)**

| Sample | Processing method | Experimental process | crystal form | DSC peak |
|---|---|---|---|---|
| Form III | Grinding for 10min | 1g sample of the crystal form III of the compound represented by formula (I) was ground in a mortar for 10min under the protection of nitrogen. | Form III | 226.91 °C |
| Form III | Heating at 80°C for 3hrs | 1g sample of the crystal form III of the compound represented by formula (I) was laid flat, and heated at 80°C for 3h. | Form III | 226.57 °C |
| Form III | Tableting | The sample of the crystal form III of the compound represented by formula (I) was pressed into tablets. | Form III | 226.58 °C |

### Experimental conclusion:

According to the data in Table 3, the crystal form III of the compound represented by formula (I) stays unchanged under the conditions of grinding, high-temperature heating and tableting treatment, which indicates that the stability of the crystal form III of the present invention was relatively high.

### Embodiment 14 Hygroscopicity study of crystal form III of the present invention

By adopting TAQ5000VSA, the humidity was 10-90% at 25°C, stepping was 10%, and the judgment standard was that the mass change was less than 0.01% within 10000 min, and the cycle was run twice.

### Experimental results

**Table 4. Test Results of hygroscopicity of crystal Form III of the present invention**

| Test sample | 10.0%RH-80.0%RH | crystal type |
|---|---|---|
| Form III | 0.9332% (slightly hygroscopic) | Not changed |

### Experimental conclusion:

As can be seen from table 4, the water absorption of the sample of crystal form III of the compound represented by formula (I) increases with the increase of humidity between 10.0% RH and 80.0% RH under the condition of 25°C, the weight change is 0.9332%, less than 15% but no less than 2%, and the sample was slightly hygroscopic; the desorption process of the sample coincides with the adsorption process during the humidity change ranging from 0% to 85%.

### Embodiment 15 Pharmacokinetic experiment of the crystal form III of the present invention and the free state in Rats

SD rats were used as test animals, LC/MS/MS method was employed to measure the drug concentration in plasma at different time points after the SD rats were gavaged with the crystal form III and free state of the compound represented by formula (I), to study the pharmacokinetic behavior of the crystal form III and free state of the compound represented by formula (I) in SD rats, and to evaluate its pharmacokinetic characteristics.

Test samples: crystal form III of the compound represented by formula (I) (see embodiment 1 for its preparation method) and free state (prepared according to the method described in patent application WO2016091042A1).

Experimental animals: eight healthy SD rats, half male and half female, were divided into 2 groups, purchased from Sippr-BK Experimental Animal Co., Ltd. with animal production license number SCXK (Shanghai) 2008-0016.

Drug preparation: 0.5% CMC-Na, a uniform suspension was prepared by ultrasound with a preparation concentration of 0.5 mg/mL for oral administration.

Administration: eight healthy SD rats, half male and half female, were administered gavagedly after fasting for one night with a volume of 10 mL/kg.

### Methods:

Eight healthy SD rats, half male and half female, were administered gavagedly after fasting overnight. 0.1mL blood was collected through jugular vein puncture before and 0.5, 1, 2, 4, 6, 8, 12, 24h after the administration, heparin sodium was used for anticoagulation, and plasma was separated by centrifugation at 3500 rpm for 10min and stored at -20°C. LC/MS/MS was used to determine the content of the compound to be detected in plasma of SD rats after gavage administration of compound.

### Experimental results

**Table 5, Pharmacokinetic evaluation results in SD rats (po: 5.0 mg/kg)**

| Test sample | T_{1/2}(h) | AUCₗₐₛₜ (ng/mL×h) | Cl/F_{_obs} (mL/min/kg) | Vz/F_ _{obs} (mL/kg) |
|---|---|---|---|---|
| Form III | 4.21 | 12618 | 6.70 | 2453 |
| free state | 3.62 | 9283 | 9.82 | 3019 |

Wherein, T_{1/2} is half-life period; AUCₗₐₛₜ is the area under the curve 0 → t during administration; Cl/F is the clearance rate; Vz/F is the apparent distribution volume.

### Experimental conclusion:

As can be seen from table 5, compared with free state, the crystal form III of the compound represented by formula (I) has a longer half-life period, lower clearance rate, higher exposure amount, indicating that the crystal form III of the compound represented by formula (I) has good pharmacokinetic properties.

## Claims

1. A crystal form III of the compound represented by formula (I), wherein after Cu-Kα radiation, an X-ray powder diffraction spectrum represented by 2θ diffraction angles is obtained, in which there are characteristic peaks at 3.88, 7.54, 14.76, 18.64 and 22.21,

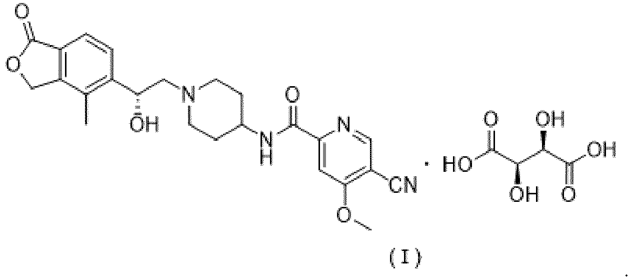

2. The crystal form III as defined in claim 1, wherein the crystal form III has characteristic peaks at 3.88, 7.54, 11.22, 14.76, 17.29, 18.64, 20.28, 22.21, 23.79, 25.34 and 27.09.

3. The crystal form III as defined in claim 1, wherein the crystal form III has characteristic peaks at 3.88, 7.54, 11.22, 11.61, 12.26, 12.73, 13.35, 13.64, 14.76, 15.98, 16.47, 17.07, 17.29, 18.64, 20.28, 20.62, 22.21, 23.16, 23.79, 24.14, 24.85, 25.34, 26.08, 26.85, 27.09, 28.77, 29.74, 32.22, 33.66, 34.50, 35.60, 37.42 and 39.27.

4. The crystal form III as defined in any one of claims 1-3, wherein the error range of 2θ angle is ±0.2.

5. A method for preparing crystal form III as defined in any one of claims 1-4, which is one of the following methods:
Method (1): reacting the free state of the compound represented by formula (I) with L-tartaric acid in one solvent or a mixed solvent, then after stirring, crystallizing, filtering and drying, the target crystal form III is obtained; the solvent is a sulfoxide solvent, an amide solvent or an alcohol solvent, and the mixed solvent is a mixed solvent of a sulfoxide solvent and an alcohol solvent, or a mixed solvent of an amide solvent and an alcohol solvent; the sulfoxide solvent is preferably dimethyl sulfoxide, the amide solvent is preferably N,N-dimethylformamide or N,N-dimethylacetamide, and the alcohol solvent is preferably methanol, ethanol, *n*-propanol, *iso*-propanol or *n*-butanol;
Method (2): dissolving the compound represented by formula (I) in a solvent or a mixed solvent, then after crystallizing, filtering and drying, the target crystal form III is obtained; the crystallizing is conducted at room temperature, or while cooling or volatilizing the solvent or induced by crystal seed, and the temperature for the cooling is -10 to 25°C, preferably the crystallizing is conducted at room temperature; the solvent is a sulfoxide solvent, an amide solvent or an alcohol solvent, and the mixed solvent is a mixed solvent of a sulfoxide solvent and an alcohol solvent, or a mixed solvent of an amide solvent and an alcohol solvent; the sulfoxide solvent is preferably dimethyl sulfoxide, the amide solvent is preferably N,N-dimethyl formamide or N,N-dimethyl acetamide, and the alcohol solvent is preferably methanol, ethanol, *n*-propanol, *iso-*propanol or *n*-butanol, the mixed solvent is more preferably dimethyl sulfoxide/methanol, dimethyl sulfoxide/ethanol, dimethyl sulfoxide/*n*-propanol, dimethyl sulfoxide/*iso*-propanol, dimethyl sulfoxide/*n*-butanol, N,N-dimethyl formamide/ethanol or N,N-dimethyl acetamide/ethanol.

6. A pharmaceutical composition comprising the crystal form III as defined in any one of claims 1-4 and a pharmaceutically acceptable carrier, a diluent or an excipient.

7. A method for preparing the pharmaceutical composition, wherein the method comprises mixing the crystal form III as defined in any one of claims 1-4 with a pharmaceutically acceptable carrier, a diluent or a excipient.

8. A use of the crystal form III as defined in any one of claims 1-4 or the pharmaceutical composition as defined in claim 6 in the manufacture of a medicament for treating and/or preventing a disease or a condition related to renal outer medullary potassium channel (ROMK) inhibition, the disease or condition is preferably hypertension or heart failure.
